# EUROPEAN PATENT SPECIFICATION

(11) **EP 2 736 499 B1**
(45) Date of publication and mention of the grant of the patent: **11.05.2016**
(21) Application number: 12817601.3
(22) Date of filing: 20.07.2012
(51) Int. Cl.: A61K 9/22, A61K 9/20, A61K 31/197, A61K 47/38

(54) **SUSTAINED RELEASE TABLET COMPRISING PREGABALIN THROUGH TWO-PHASE RELEASE-CONTROLLING SYSTEM**
TABLETTE MIT VERZÖGERT FREIGESETZTEM PREGABALIN DURCH EIN ZWEIPHASEN-FREISETZUNGSSTEUERUNGSSYSTEM
COMPRIMÉ À LIBÉRATION CONTINUE COMPRENANT DE LA PRÉGABALINE PAR UN SYSTÈME DE COMMANDE DE LIBÉRATION À DEUX PHASES

(30) Priority: 26.07.2011 KR 20110074011
(43) Date of publication of application: 04.06.2014
(73) Proprietor: Yuhan Corporation, Seoul 156-754 (KR)
(72) Inventor: WOO, Jeong-Hoon, Suwon-si Gyeonggi-do 440-320 (KR); NA, Woon-Sook, Suwon-si Gyeonggi-do 443-470 (KR); JEONG, Yang-Soo, Yongin-si Gyeonggi-do 446-905 (KR); HYUN, Chang-Keun, Suwon-si Gyeonggi-do 443-706 (KR); PARK, Yoong-Sik, Yongin-si Gyeonggi-do 448-160 (KR)
(74) Representative: Turner, Craig Robert
(86) International application number: PCT/KR2012/005831
(87) International publication number: WO 2013/015578

(56) References cited:
- WO-A1-2009/066325
- WO-A2-2007/052125
- WO-A2-2011/049309
- WO-A2-2011/053003
- US-A1- 2010 255 067

## Description

### Technical Field

The present invention relates to a sustained release tablet having two-phase release-controlling system, which consists of a first release-controlling phase comprising pregabalin or its salt and hydroxypropyl methylcellulose; and a second release-controlling phase comprising polyethylene oxide as a swelling polymer, the first release-controlling phase being homogeneously dispersed in the second release-controlling phase.

### Background Art

Pregabalin, chemically known as (S)-(+)-3-aminomethyl-5-methylhexanoic acid, binds to the alpha-2-delta subunit of calcium channels and is related to the endogenous inhibitory neurotransmitter γ-aminobutync acid (GABA), which is involved in the regulation of brain neuronal activity. Pregabalin is useful for treating epilepsy, neuropathic pain, fibromyalgia, etc. Pregabalin is being marketed as an immediate release (IR) formulation, e.g., capsules containing 75 mg, 150 mg, or 300 mg and is usually administered to patients twice a day.

The conversion of drugs administered more than twice a day into a form for oncea-day administration can improve patients' drug compliance; reduce any side effects originated from maximum blood concentration exceeding the desired effective blood concentration and/or sudden increase of blood concentration; and increase the maintenance time of effective blood concentration, thereby increasing pharmacological effects. However, there are some problems in designing a pregabalin-containing formulation for once a day administration. Because pregabalin is absorbed through L-amino acid transport system, it does not exhibit uniform gastrointestinal absorptions. And also, because the absorption of pregabalin takes place in the upper small intestine where most of L-amino acid transporters reside, an average absorption window thereof is less than 6 hours (Su T-Z, Feng MR, Weber ML: Mediation of highly concentrative uptake of pregabalin by L-type amino acid transport in Chinese hamster ovary and Caco-2 cells. J Pharmacol Exp Ther 2005, 313:1406-1415). Therefore, it is very difficult to design pregabalin-containing formulations for once-a-day administration, which can show a release profile equivalent to the formulation for twice-a-day administration, according to conventional technologies for sustained release formations. Gastro-retentive drug delivery systems may be considered as one of the methods for designing a sustained release formulation for pregabalin. The gastro-retentive drug delivery systems are classified into three systems, i.e., an expansion-by-swelling system, a floating or buoyant system, and a bioadhesive system. As a gastro-retentive drug delivery system, WO 2007/052125 has disclosed a sustained release formulation for once daily oral administration comprising pregabalin, a matrix forming agent, and a swelling agent. However, since the formulation disclosed in WO 2007/052125 is designed for inducing the gastro-retention by simple swelling, individual variations in the absorption of pregabalin (especially, time to reach maximum plasma concentration, Tmax) is very high according to patients' respective gastrointestinal conditions, e.g., according to patients' respective gastrointestinal motilities. And also, there cannot be excluded the possibility to pass through the pylorus depending on patients' gastrointestinal conditions, thereby resulting in not showing sustained release patterns. In addition, there is a disadvantage that matrix hardness of the formulation becomes decreased at the time of about 24 hours.

WO 2006/078811 has been disclosed a controlled release formulation of gabapentin and pregabalin, comprising an immediate release component, a sustained release component, and a delayed release component. However, since the formulation is not a gastro-retentive form, there is a problem that it releases pregabalin not only in the upper small intestine (major absorption site of pregabalin), but also in other gastrointestinal sites such as the stomach and the lower small intestine.

### Disclosure of Invention

### Technical Problem

The present inventors performed various researches for developing a sustained release formulation comprising pregabalin having limited absorption window as an active ingredient. Especially, the present inventors carried out various researches for designing a sustained release formulation in a gastro-retentive form, which exhibits a controlled release profile capable of minimizing individual variations. We newly designed a two-phase release-controlling system, in which a first release-controlling phase for both controlling the release of pregabalin and promoting floatability is homogeneously dispersed in a second release-controlling phase having both swellability and floatability. We found that the two-phase release-controlling system can stably show both 'swellability' an 'floatability'; and thus accomplish effective gastro-retention, which make it possible to minimize the individual variations originated from e.g., different gastrointestinal motilities.

Therefore, it is an object of the present invention to provide a sustained release formulation comprising pregabalin or its salt through two-phase release-controlling system.

### Solution to Problem

In accordance with an aspect of the present invention, there is provided a sustained release tablet having two-phase release-controlling system, which consists of a first release-controlling phase comprising pregabalin or its salt and hydroxypropyl methylcellulose; and a second release-controlling phase comprising polyethylene oxide as a swelling polymer, the first release-controlling phase being homogeneously dispersed in the second release-controlling phase.

In the first release-controlling phase, the polyethylene oxide may have an average molecular weight ranging from 100,000 to 7,000,000; and be present in an amount ranging from 10 to 70 % by weight, based on the total weight of the tablet.

In the first release-controlling phase, the hydroxypropyl methylcellulose may have a viscosity ranging from 100 to 150,000 centipoises; and be present in an amount ranging from 5 to 40 % by weight, based on the total weight of the tablet.

The first release-controlling phase may further comprise hydroxypropyl cellulose as a binder; and the second release-controlling phase may further comprise crospovidone or sodium starch glycolate as a supplemental floating agent.

In an embodiment, a time to reach maximum plasma concentration (Tmax) may range from 4 to 8 hours after oral administration of the sustained release tablet of the present invention. In another embodiment, the sustained release tablet of the present invention may be magnified to 12 mm of size when contacting with water. In still another embodiment, the sustained release tablet of the present invention may be floated within 30 minutes after contact with water and the resultant floating state may be maintained for at least 12 hours.

In accordance with another of the present invention, there is provided a process for preparing a sustained release tablet having two-phase release-controlling system, the process comprising (a) granulating a mixture of pregabalin or its salt and hydroxypropyl methylcellulose; and (b) mixing the granules obtained in the step (a) with polyethylene oxide and a pharmaceutically acceptable excipient, followed by compressing the resulting mixture.

In the process of the present invention, the granulating may be performed using a binder solution.

### Advantageous Effects of Invention

The sustained release tablet according to the present invention can effectively control the release of pregabalin along with showing controlled and prolonged release profiles (i.e., without showing initial sudden release thereof) at the time of oral administration. In the sustained release tablet of the present invention, the release of pregabalin is controlled in the first release-controlling phase; and then the released pregabalin from the first release-controlling phase is further controlled during the pass through the second release-controlling phase. That is, in the sustained release tablet of the present invention, the release of pregabalin is controlled by two phase release-controlling system. The sustained release tablet according to the present invention may be administered once a day by controlling the release mechanism of pregabalin having limited absorption window. Especially, the polymer in the first release-controlling phase can enhance floatability effectively and thus ensure both 'swellability' and 'floatability' which increases the shape-maintenance time as well as floating-maintenance time after contact with water, thereby minimizing individual variations in the absorption of pregabalin (especially, time to reach maximum plasma concentration, Tmax) originated from different gastrointestinal motilities. And also, it is possible to control a maximum blood concentration (Cmax) and a bioavailability by controlling the release time and/or the gastro-retentive characteristics through varying amounts of the polymer in the first and/or second release-controlling phase(s).

### Brief Description of Drawings

FIG. 1 shows the appearances of the sustained release tablet prepared according to the present invention and the sustained release tablet of Comparative Example 1, which were obtained 6 hours after initiating the dissolution test. A: the sustained release tablet prepared according to the present invention, B: the sustained release tablet of Comparative Example 1.
FIG. 2 shows the results of pharmacokinetic studies of the sustained release tablets prepared according to the present invention (Examples 5, 13, and 20) and the comparative formulation.
FIG. 3 shows the results of pharmacokinetic studies of the sustained release tablets prepared according to the present invention (Examples 23, 28, and 31) and the comparative formulation.

### Best Mode for Carrying out the Invention

The present invention provides a sustained release tablet having two-phase release-controlling system, which consists of a first release-controlling phase comprising pregabalin or its salt and hydroxypropyl methylcellulose; and a second release-controlling phase comprising polyethylene oxide as a swelling polymer, the first release-controlling phase being homogeneously dispersed in the second release-controlling phase.

The sustained release tablet according to the present invention is a gastro-retentive drug delivery system, wherein the drug (i.e., pregabalin) is continuously released while it is staying in the stomach for a predetermined time after oral administration, thereby maximizing the absorption of pregabalin in the upper small intestine. In the sustained release tablet of the present invention, the release of pregabalin is controlled in the first release-controlling phase; and then the released pregabalin from the first release-controlling phase is further controlled during the pass through the second release-controlling phase. That is, in the sustained release tablet of the present invention, the release of pregabalin is controlled by two phase release-controlling system. And also, the polymer in the first release-controlling phase can enhance floatability effectively and thus ensure both 'swellability' and 'floatability' which increases the shape-maintenance time as well as floating-maintenance time after contact with water, thereby minimizing individual variations in the absorption of pregabalin (especially, time to reach maximum plasma concentration, Tmax) originated from different gastrointestinal motilities. In addition, it is possible to control a maximum blood concentration (Cmax) and a bioavailability by controlling the release time and/or the gastro-retentive characteristics through varying amounts of the polymer in the first and/or second release-controlling phase(s). Therefore, the sustained release tablet according to the present invention may be administered once a day by controlling the release mechanism of pregabalin having limited absorption window.

The first release-controlling phase comprises hydroxypropyl methylcellulose, so as to control the release of pregabalin and improve floatability. The hydroxypropyl methylcellulose expands on contact with water to form a matrix, thereby controlling the release of pregabalin; and improves floatability based on its own low density. The hydroxypropyl methylcellulose used in the sustained release tablet of the present invention may have a viscosity ranging from 100 to 150,000 centipoises, preferably 400 to 100,000 centipoises. The hydroxypropyl methylcellulose may be present in an amount ranging from 10 to 70 % by weight, based on the total weight of the tablet. When the hydroxypropyl methylcellulose is used in an amount below 10 %, dissolution of the active ingredient may be too fast to show a desired sustained release pattern. When the hydroxypropyl methylcellulose is used in an amount above 70 %, dissolution of the active ingredient may become slow due to thick matrix formation, which results in showing a drug concentration below an appropriate therapeutic concentration. The first release-controlling phase may further comprise a binder conventionally used in the field of pharmaceutics, preferably hydroxypropyl cellulose. An amount of the binder is not limited. That is, the binder may be used in an amount sufficient for forming the first release-controlling phase, for example the first release-controlling phase in a granular form.

The second release-controlling phase comprises polyethylene oxide as a swelling polymer, so as to improve both swellability and floatability for increasing the gastro-retention time. The polyethylene oxide may have an average molecular weight ranging from 100,000 to 7,000,000, preferably 4,000,000 to 7,000,000. The polyethylene oxide may be present in an amount ranging from 5 to 40 % by weight, based on the total weight of the tablet. When the polyethylene oxide is used in an amount below 5 %, sufficient swelling may not be shown. When the polyethylene oxide is used in an amount above 40 %, it may be difficult to control a release of the drug.

The second release-controlling phase may comprise one or more excipient conventionally used in the field of pharmaceutics, such as a diluent, a lubricant, etc. For example, the diluent includes microcrystalline cellulose, lactose, etc.; and the lubricant includes magnesium stearate, zinc stearate, colloidal silicon dioxide, etc. And also, the second release-controlling phase may further comprise crospovidone or sodium starch glycolate as a supplemental floating agent. If necessary, the second release-controlling phase may further comprise hydroxypropyl methylcellulose as an erosion-preventing agent, in order to prevent potential erosion after swelling.

The sustained release tablet according to the present invention may be coated with a conventional film coating agent.

In an embodiment, a time to reach maximum plasma concentration (Tmax) may range from 4 to 8 hours after oral administration of the sustained release tablet of the present invention. In another embodiment, the sustained release tablet of the present invention may be magnified to 12 mm of size when contacting with water. In still another embodiment, the sustained release tablet of the present invention may be floated within 30 minutes after contact with water and the resultant floating state may be maintained for at least 12 hours. In still another embodiment, when an *in vitro* dissolution test of the sustained release tablet of the present invention is carried out according to the Korean Pharmacopeia or the US Pharmacopeia, the active ingredient (i.e., pregabalin) is released in an amount below 30% within 1 hour, in an amount (cumulative dissolution rate) ranging 20 to 75% within 4 to 6 hours; and an amount (cumulative dissolution rate) above 75% within 12 hours.

The present invention also provides a process for preparing a sustained release tablet having two-phase release-controlling system. The sustained release tablet having two-phase release-controlling system according to the present invention may be prepared by providing a first release-controlling phase, typically a first release-controlling phase in a granular form, and then compressing the first release-controlling phase with the remaining components. For example, the process for preparing a sustained release tablet having two-phase release-controlling system may comprise (a) granulating a mixture of pregabalin or its salt and hydroxypropyl methylcellulose; and (b) mixing the granules obtained in the step (a) with polyethylene oxide and a pharmaceutically acceptable excipient, followed by compressing the resulting mixture.

The granulating may be performed using a binder solution. In an embodiment, the step (a) may be performed by granulating a mixture of pregabalin (or its salt) and hydroxypropyl methylcellulose in a wet granulator (e.g., High Shear Mixer or One-pot), using a binder solution (e.g., an aqueous ethanol solution of hydroxypropyl cellulose), to form a first release-controlling phase. The obtained first release-controlling phase is mixed with components of the second release-controlling phase, i.e., polyethylene oxide and a pharmaceutically acceptable excipient, followed by compressing the resulting mixture. The pharmaceutically acceptable excipient includes a diluent, a lubricant (such as magnesium stearate, zinc stearate, colloidal silicon dioxide, etc.), etc. And also, the components of the second release-controlling phase may further comprise crospovidone or sodium starch glycolate as a supplemental floating agent; and/or hydroxypropyl methylcellulose as an erosion-preventing agent. The process of the present invention may further comprise coating with a conventional film coating agent.

The present invention will be described in further detail with reference to the following examples and experimental examples. These examples and experimental examples are for illustrative purposes only and are not intended to limit the scope of the present invention.

### Examples 1 to 34

Sustained release tablets were prepared according to the components and amounts shown in Tables 1 to 5. The amounts of Tables 1 to 5 represent the weight (mg) of each component per one tablet. Pregabalin and hydroxypropyl methylcellulose (Metolose™ 90SH-100,000cps SR, Shinetsu) were charged in a high speed mixer and then a binder solution (prepared by dissolving hydroxypropyl cellulose (HPC JF) in a 50% ethanol solution) was added thereto. The mixer was rotated at 250 rpm for 3 minutes to give granules. The resulting granules were dried and then sieved with a milling machine. The obtained granules were mixed with polyethylene oxide (Polyox 301 and/or Polyox 303), along with a supplementary floating agent (crospovidone or sodium starch glycolate), a diluent (hydroxypropyl cellulose or microcrystalline cellulose), or a lubricant (colloidal silicon dioxide) for 5 minutes. Magnesium stearate was additionally mixed with the mixture for 3 minutes, followed by compressing to obtain sustained release tablets.

**<Table 1>**

| Components | | Example (mg per one tablet) | | | | | | | | |
|---|---|---|---|---|---|---|---|---|---|---|
| | | 1 | 2 | 3 | 4 | 5 | 6 | 7 | 8 | 9 |
| First release-controlling phase | Pregabalin | 300 | 300 | 300 | 300 | 300 | 300 | 300 | 300 | 300 |
| | Hydroxypropyl methylcellulose | 350 | 350 | 350 | 350 | 300 | 250 | 300 | 300 | 300 |
| | Hydroxypropyl cellulose | 25 | 25 | 25 | 25 | 25 | 25 | 25 | 25 | 25 |
| | Polyethylene oxide (Polyox 301) | 100 | - | 100 | 100 | 100 | 100 | 50 | 150 | 100 |
| Second release-controlling phase | Polyethylene oxide (Polyox 303) | - | 100 | - | - | - | - | - | - | - |
| | Crospovidone | - | - | 50 | - | 50 | 50 | 50 | 50 | 100 |
| | Sodium starch glycolate | - | - | - | 50 | - | - | - | - | - |
| | Colloidal silicon dioxide | 15 | 15 | 15 | 15 | 15 | 15 | 15 | 15 | 15 |
| | Magnesium stearate | 5 | 5 | 5 | 5 | 5 | 5 | 5 | 5 | 5 |
| Total | | 795 | 795 | 845 | 845 | 795 | 745 | 745 | 845 | 845 |

**<Table 2>**

| Components | | Example (mg per one tablet) | | | | | | | | |
|---|---|---|---|---|---|---|---|---|---|---|
| | | 10 | 11 | 12 | 13 | 14 | 15 | 16 | 17 | 18 |
| First release-controlling phase | Pregabalin | 300 | 300 | 300 | 300 | 300 | 300 | 300 | 300 | 300 |
| | Hydroxypropyl methylcellulose | 250 | 150 | 150 | 350 | 350 | 100 | 100 | 250 | 250 |
| | Hydroxypropyl cellulose | 25 | 15 | 15 | 25 | 25 | 10 | 10 | 15 | 15 |
| | Polyethylene oxide (Polyox 301) | 100 | 300 | 200 | 100 | 100 | 100 | 100 | 100 | 100 |
| | Hyd roxypropyl methylcellulose (100,000 cps) | 100 | - | - | - | - | - | - | - | - |
| Second release-controlling phase | Hydroxypropyl methylcellulose (400 cps) | - | - | 100 | - | - | 300 | - | - | - |
| | Crospovidone | - | 50 | 50 | - | - | 50 | 50 | - | - |
| | Sodium starch glycolate | - | - | - | 50 | - | - | - | 100 | 200 |
| | Microcrystalline cellulose | - | - | - | 50 | 100 | - | - | - | - |
| | Colloidal silicon dioxide | 15 | 15 | 15 | 15 | 15 | 15 | 15 | 15 | 15 |
| | Magnesium stearate | 5 | 5 | 5 | 5 | 5 | 5 | 5 | 5 | 5 |
| Total | | 845 | 835 | 835 | 895 | 895 | 880 | 580 | 785 | 885 |

**<Table 3>**

| Components | | Example (mg per one tablet) | | | |
|---|---|---|---|---|---|
| | | 19 | 20 | 21 | 22 |
| First release-controlling phase | Pregabalin | 300 | 300 | 300 | 300 |
| | Hydroxypropyl methylcellulose | 150 | 200 | 150 | 150 |
| | Hydroxypropyl cellulose | 15 | 20 | 15 | 15 |
| | Polyethylene oxide (Polyox 301) | 100 | 100 | 100 | 100 |
| Second release-controlling phase | Hydroxypropyl methylcellulose(100 cps) | - | - | 100 | - |
| | Crospovidone | - | - | - | 50 |
| | Colloidal silicon dioxide | 15 | 15 | 15 | 15 |
| | Magnesium stearate | 5 | 5 | 5 | 5 |
| Total | | 585 | 640 | 685 | 635 |

**<Table 4>**

| Components | | Example (mg per one tablet) | | | | | | | | |
|---|---|---|---|---|---|---|---|---|---|---|
| | | 23 | 24 | 25 | 26 | 27 | 28 | 29 | 30 | 31 |
| First release-controlling phase | Pregabalin | 150 | 150 | 150 | 150 | 150 | 150 | 150 | 150 | 150 |
| | Hydroxypropyl methylcellulose | 300 | 300 | 450 | 350 | 350 | 500 | 200 | 350 | 400 |
| | Hydroxypropyl cellulose | 25 | 25 | 25 | 25 | 25 | 25 | 20 | 20 | 20 |
| | Polyethylene oxide (Polyox 301) | 100 | 100 | 100 | 100 | 100 | 100 | 100 | 100 | 100 |
| Second release-controlling phase | Crospovidone | 50 | 50 | 50 | - | - | - | - | - | - |
| | Sodium starch glycolate | - | - | - | 50 | 50 | 50 | - | - | - |
| | Microcrystalline cellulose | - | 150 | - | 50 | 200 | 50 | - | - | - |
| | Colloidal silicon dioxide | 10 | 10 | 10 | 10 | 10 | 10 | 10 | 10 | 10 |
| | Magnesium stearate | 5 | 5 | 5 | 5 | 5 | 5 | 5 | 5 | 5 |
| Total | | 640 | 740 | 790 | 740 | 890 | 890 | 485 | 635 | 685 |

**<Table 5>**

| Components | | Example (mg per one tablet) | | |
|---|---|---|---|---|
| | | 32 | 33 | 34 |
| First release-controlling phase | Pregabalin | 75 | 75 | 75 |
| | Hydroxypropyl methylcellulose | 300 | 575 | 475 |
| | Hydroxypropyl cellulose | 20 | 25 | 15 |
| | Polyethylene oxide (Polyox 301) | 100 | 100 | 100 |
| Second release-controlling phase | Crospovidone | 50 | 50 | - |
| | Sodium starch glycolate | - | 50 | - |
| | Microcrystalline cellulose | - | - | - |
| | Colloidal silicon dioxide | 10 | 10 | 10 |
| | Magnesium stearate | 5 | 5 | 5 |
| Total | | 560 | 890 | 680 |

### Comparative Example 1

Pregabalin (300 g), Kollidon SR (256 g), Plasdone XL (280 g), Polyox N60K NF (224 g), and Carbopol 71G (56.5 g) were mixed for 5 minutes. Magnesium stearate (5.5 g) was additionally mixed with the mixture for 3 minutes, followed by compressing to obtain tablets.

### Experimental Example 1: In vitro dissolution test

The dissolution tests of the tablets prepared in Examples 1 to 34 were performed according to the 'Dissolution Test 2 (Paddle Method)' of the Korean Pharmacopeia. 900 ml of a 0.06N HCl solution was used as a dissolution medium and the dissolution test was performed at 37 ± 0.5 °C and at the paddle rotation speed of 50 rpm. An aliquot was taken from the dissolution medium at the time of 0.5, 1, 2, 3, 4, 6, 8, 12, 16, and 24 minutes, respectively. Each aliquot was analyzed with HPLC (at 210 nm) to calculate the dissolution rates. The results are presented in Tables 6 to 10. As shown in Tables 6 to 10, the tablets prepared according to the present invention showed excellent sustained release dissolution patterns.

**<Table 6>**

| Time (hr) | Example (Dissolution rate, %) | | | | | | | | |
|---|---|---|---|---|---|---|---|---|---|
| | 1 | 2 | 3 | 4 | 5 | 6 | 7 | 8 | 9 |
| 0.5 | 9.2 | 11.1 | 10.2 | 11.0 | 11.8 | 11.2 | 11.3 | 10.4 | 9.3 |
| 1 | 16.1 | 16.9 | 16.3 | 17.2 | 18.4 | 17.6 | 18.2 | 16.4 | 16.1 |
| 2 | 25.5 | 25.6 | 25.1 | 25.1 | 28.3 | 27.3 | 28.7 | 25.9 | 26.7 |
| 3 | 36.6 | 32.9 | 32.5 | 33.7 | 36.7 | 35.6 | 36.7 | 33.1 | 34.6 |
| 4 | 46.2 | 39.6 | 38.7 | 40.2 | 43.9 | 43.3 | 44.0 | 40.1 | 41.5 |
| 6 | 58.6 | 51.2 | 49.5 | 51.5 | 55.5 | 55.8 | 54.2 | 51.0 | 52.7 |
| 8 | 66.5 | 60.7 | 58.1 | 61.5 | 65.5 | 66.9 | 62.9 | 60.7 | 62.0 |
| 12 | 85.5 | 76.7 | 72.2 | 77.7 | 80.6 | 83.4 | 75.8 | 75.3 | 77.0 |
| 16 | 100.6 | 89.1 | 83.5 | 88.9 | 92.3 | 96.3 | 86.4 | 86.7 | 88.5 |
| 24 | 101.4 | 101.6 | 96.9 | 100.9 | 105.1 | 108.8 | 101.8 | 101.4 | 103.9 |

**<Table 7>**

| Time (hr) | Example (Dissolution rate, %) | | | | | | | | |
|---|---|---|---|---|---|---|---|---|---|
| | 10 | 11 | 12 | 13 | 14 | 15 | 16 | 17 | 18 |
| 0.5 | 8.2 | 9.2 | 9.3 | 11.9 | 11.5 | 9.1 | 11.6 | 11.0 | 11.9 |
| 1 | 13.1 | 14.4 | 15.5 | 17.9 | 17.6 | 14.7 | 18.3 | 17.2 | 19.9 |
| 2 | 21.3 | 23.0 | 25.1 | 27.9 | 26.9 | 24.1 | 29.7 | 26.6 | 30.2 |
| 3 | 28.3 | 30.3 | 33.5 | 35.8 | 34.1 | 31.5 | 39.3 | 34.0 | 38.1 |
| 4 | 34.8 | 37.1 | 40.6 | 41.4 | 40.3 | 38.7 | 47.9 | 41.4 | 46.1 |
| 6 | 47.1 | 49.7 | 54.2 | 53.1 | 51.3 | 50.6 | 62.5 | 53.7 | 59.8 |
| 8 | 57.5 | 60.5 | 65.1 | 62.0 | 60.3 | 59.6 | 73.9 | 64.4 | 70.5 |
| 12 | 74.9 | 77.0 | 82.4 | 77.2 | 75.1 | 75.0 | 91.5 | 80.6 | 84.1 |
| 16 | 87.4 | 89.1 | 93.8 | 88.3 | 86.0 | 87.0 | 99.8 | 91.5 | 94.0 |
| 24 | 103.0 | 104.7 | 103.8 | 98.9 | 96.6 | 99.4 | 102.6 | 102.2 | 102.7 |

**<Table 8>**

| Time (hr) | Example (Dissolution rate, %) | | | |
|---|---|---|---|---|
| | 19 | 20 | 21 | 22 |
| 0.5 | 10.2 | 9.7 | 8.9 | 9.8 |
| 1 | 15.5 | 15.0 | 14.3 | 16.2 |
| 2 | 24.8 | 24.1 | 23.3 | 26.6 |
| 3 | 33.2 | 32.3 | 31.4 | 35.3 |
| 4 | 40.7 | 39.7 | 38.7 | 43.2 |
| 6 | 54.1 | 52.8 | 52.2 | 56.9 |
| 8 | 66.1 | 64.4 | 63.5 | 69.1 |
| 12 | 84.1 | 80.3 | 81.5 | 87.0 |
| 16 | 95.3 | 92.3 | 93.0 | 98.0 |
| 24 | 102.8 | 101.0 | 103.4 | 106.5 |

**<Table 9>**

| Time (hr) | Example (Dissolution rate, %) | | | | | | | | |
|---|---|---|---|---|---|---|---|---|---|
| | 23 | 24 | 25 | 26 | 27 | 28 | 29 | 30 | 31 |
| 0.5 | 12.9 | 12.7 | 11.5 | 13.1 | 12.7 | 12.3 | 12.8 | 12.5 | 11.0 |
| 1 | 19.1 | 19.5 | 17.7 | 19.8 | 18.8 | 18.6 | 20.2 | 19.0 | 17.4 |
| 2 | 30.1 | 29.5 | 27.2 | 30.2 | 28.1 | 28.0 | 33.9 | 29.1 | 26.7 |
| 3 | 39.3 | 36.6 | 34.0 | 39.0 | 35.9 | 35.5 | 43.4 | 37.2 | 34.4 |
| 4 | 47.1 | 43.5 | 40.8 | 46.2 | 42.5 | 41.6 | 51.9 | 44.3 | 43.2 |
| 6 | 58.7 | 50.8 | 50.2 | 57.5 | 53.6 | 51.1 | 66.4 | 56.4 | 51.7 |
| 8 | 68.9 | 58.1 | 56.6 | 67.9 | 63.1 | 58.7 | 78.4 | 66.3 | 62.0 |
| 12 | 83.9 | 70.6 | 69.7 | 83.5 | 77.8 | 71.6 | 95.1 | 81.6 | 76.0 |
| 16 | 95.4 | 80.6 | 79.8 | 94.3 | 87.9 | 82.1 | 104.7 | 92.6 | 87.4 |
| 24 | 107.9 | 95.1 | 94.8 | 109.0 | 101.0 | 97.0 | 105.7 | 104.4 | 99.1 |

**<Table 10>**

| Time | Example (Dissolution rate, %) | | |
|---|---|---|---|
| (hr) | 32 | 33 | 34 |
| 0.5 | 11.0 | 10.7 | 11.6 |
| 1 | 18.9 | 16.3 | 17.8 |
| 2 | 30.5 | 24.7 | 27.6 |
| 3 | 38.3 | 31.1 | 36.7 |
| 4 | 45.9 | 36.3 | 43.2 |
| 6 | 58.3 | 44.2 | 53.4 |
| 8 | 66.6 | 51.3 | 60.5 |
| 12 | 79.2 | 63.1 | 75.4 |
| 16 | 88.3 | 73.1 | 85.3 |
| 24 | 96.4 | 86.7 | 97.1 |

### Experimental Example 2: Measurement of floating-initiation times and floating-maintenance times

The dissolution tests of the tablets prepared in the above Examples were performed according to the 'Dissolution Test 2 (Paddle Method)' of the Korean Pharmacopeia. 900 ml of a 0.06N HCl solution was used as a dissolution medium and the dissolution test was performed at 37 ± 0.5 °C and at the paddle rotation speed of 50 rpm. The floating lag times were measured and then the floating-maintenance times were measured until 24 hours. The results are presented in Tables 11 to 13. As shown in Tables 11 to 13, the tablets prepared according to the present invention initiated floating within 30 minutes; and each floating was maintained for at least 24 hours.

**<Table 11>**

| | Example 5 | Example 13 | Example 20 |
|---|---|---|---|
| Floating lag time | 3 ∼ 6 min. | 20 ∼ 25 min. | 12 ∼ 15 min. |
| Floating-maintenance time | 24 hours | 24 hours | 24 hours |

**<Table 12>**

| | Example 23 | Example 28 | Example 31 |
|---|---|---|---|
| Floating lag time | 3 ∼ 6 min. | 18 ∼ 20 min. | 8 ∼ 10 min. |
| Floating-maintenance time | 24 hours | 24 hours | 24 hours |

**<Table 13>**

| | Example 32 | Example 33 | Example 34 |
|---|---|---|---|
| Floating lag time | 4 ∼ 10 min. | 4 ∼ 7 min. | 6 ∼ 7 min. |
| Floating-maintenance time | 24 hours | 24 hours | 24 hours |

### Experimental Example 3: Measurement of swelling size

The dissolution tests of the tablets prepared in the above Examples were performed according to the 'Dissolution Test 2 (Paddle Method)' of the Korean Pharmacopeia. 900 ml of a 0.06N HCl solution was used as a dissolution medium and the dissolution test was performed at 37 ± 0.5 °C and at the paddle rotation speed of 50 rpm. Each tablet was recovered 24 hours after initiating the dissolution test and then the size thereof was measured. The results are presented in Tables 14 to 16. As shown in Tables 14 to 16, the sizes of the tablets prepared according to the present invention were increased to above 12 mm, the size of which allows for gastro-retention.

**<Table 14>**

| | Example 5 | Example 13 | Example 20 |
|---|---|---|---|
| Tablet size | 30 X 20 mm | 32 X 22 mm | 25 X 15 mm |

**<Table 15>**

| | Example 23 | Example 28 | Example 31 |
|---|---|---|---|
| Tablet size | 28 X 18 mm | 30 X 20 mm | 28 X 15 mm |

**<Table 16>**

| | Example 32 | Example 33 | Example 34 |
|---|---|---|---|
| Tablet size | 32 X 13 mm | 29 X 15 mm | 30 X 15 mm |

### Experimental Example 4: Comparison of tablet size and water-contents

The dissolution tests of the tablets prepared in Examples 5, 13, 20, and Comparative Example 1 were performed according to the 'Dissolution Test 2 (Paddle Method)' of the Korean Pharmacopeia. 900 ml of a 0.06N HCl solution was used as a dissolution medium and the dissolution test was performed at 37 ± 0.5 °C and at the paddle rotation speed of 50 rpm. Each tablet was recovered 6 hours after initiating the dissolution test and then the picture was taken. And also, each tablet was recovered 2, 6, and 24 hours after initiating the dissolution test and then the dissolution medium was removed for about 1 minute. Each size change was measured and each weigh change was obtained by measuring the water content thereof. The results are presented in
Table 17.

**<Table 17>**

| Time (h) | Example 5 | | Example 13 | | Example 20 | | Comparative Example 1 | |
|---|---|---|---|---|---|---|---|---|
| | Weight change (%) | Size change (%) | Weight change (%) | Size change (%) | Weight change (%) | Size change (%) | Weight change (%) | Size change (%) |
| 2 | 242 | 152 | 283 | 168 | 222 | 140 | 208 | 132 |
| 6 | 322 | 184 | 363 | 208 | 305 | 165 | 242 | 139 |
| 24 | 437 | 219 | 482 | 242 | 402 | 187 | Tablet crushed | Tablet crushed |

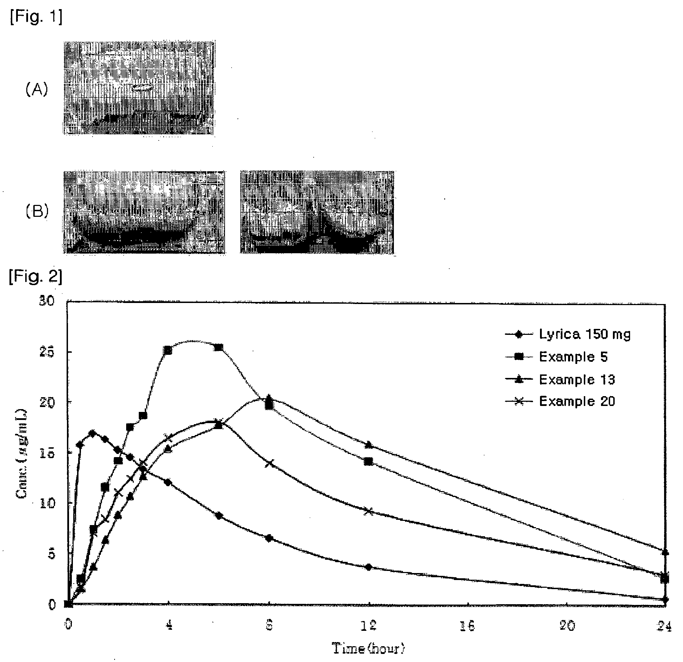

As shown in Table 17, the tablets prepared in Examples 5, 13, and 20 showed more excellent properties in size change and water contents, in comparison with the tablet of Comparative Example 1. These results shows that the tablets of the present invention can increase the gastro-retention time and thus effectively control the drug release. And also, as shown in FIG. 1, the tablet prepared according to the present invention (the tablet of Example 5) was homogeneously swelled with forming a firm matrix, while the tablet of Comparative Example 1 was crushed to pieces, not forming a firm matrix.

### Experimental Example 5: Measurement of dissolution rate according to rotation speed

The dissolution tests of the tablets prepared in Example 5 and Comparative Example 1 were performed according to the 'Dissolution Test 2 (Paddle Method)' of the Korean Pharmacopeia. 900 ml of a 0.06N HCl solution was used as a dissolution medium and the dissolution test was performed at 37 ± 0.5 °C and at the paddle rotation speeds of 50 rpm and 200 rpm, respectively. An aliquot was taken from the dissolution medium at the time of 1, 2, 3, 4, and 6 hours, respectively. Each aliquot was analyzed with HPLC (at 210 nm) to calculate the dissolution rates. The results are presented in Table 18.

**<Table 18>**

| Dissolution rate at 50 rpm and 200 rpm | | | | |
|---|---|---|---|---|
| Time (hr) | Example 5 | | Comparative Example 1 | |
| | 50 rpm | 200 rpm | 50 rpm | 200 rpm |
| 1 | 18.4 | 21.5 | 17.7 | 32.2 |
| 2 | 28.3 | 32.8 | 26.8 | 44.6 |
| 3 | 36.7 | 41.2 | 39.4 | 53.8 |
| 4 | 43.9 | 48.3 | 50.4 | 61.4 |
| 6 | 55.5 | 60.8 | 61.3 | 73.9 |

As shown in Table 18, the tablet of Example 5 showed small differences in the dissolution rate when the rotation speed of the paddle was increased. In contrast, the dissolution rate, especially the initial dissolution rate, was remarkably increased in the tablet of Comparative Example 1, when the rotation speed of the paddle was increased. These results show that the tablets of the present invention is less affected by the rotation speed of the paddle than the sustained release tablet known in the prior art; and thus that the tablets of the present invention is less affected by gastrointestinal motility, thereby minimizing individual variations.

### Experimental Example 6: Comparative pharmacokinetic evaluation

The comparative pharmacokinetic evaluations on the tablets prepared in Example 5 and Comparative Example 1 were performed using beagle dogs. Beagle dogs (body weight: about 10 kg) fasted for 12 hours were divided into 2 groups, each group having 4 dogs (i.e. n=4). The dogs were provided with a single mixed feed consisting of solid food and liquid nutrients. 1 hour after the feeding, the dogs of each group were orally administered with the tablet of Example 5 and the tablet of Comparative Example 1, respectively. The blood (about 0.5 mL) was collected using a heparin-treated injector, at the time of 0.5, 1, 1.5, 2, 2.5, 3, 4, 6, 8, 12, and 24 hours after the administration. The collected blood was centrifuged at 10,000 rpm for 1 minutes, and then the separated serum was stored at -20 °C for analysis. The concentration of pregabalin in the serum was analyzed with LC/MS/MS. The pharmacokinetic parameters obtained from the blood concentration profiles are presented in Table 19 below.

**<Table 19>**

| Parameters | Example 5 | Comparative Example 1 |
|---|---|---|
| AUC₀₋₂₄ₕᵣ (ug · hr/mL) | 321.3 ± 59.4 | 275.0 ± 83.1 |
| Cmax (ug/mL) | 26.6 ± 5.0 | 20.0 ± 6.8 |
| Tmax (hr) | 5.1 ± 1.1 (4.0 ∼ 8.0) | 6.1 ± 4.5 (1.5 ∼ 12.0) |

As shown in Table 19, the tablet of Comparative Example 1 showed larger variations than the tablet of Example 5. Especially, the time to reach maximum plasma concentration (Tmax) of the tablet of Example 5 was only from 4 to 8 hours, while Tmax of the tablet of Comparative Example 1 was from 1.5 to 12 hours. These results show that the tablet of the present invention is less affected by gastrointestinal motility, thereby minimizing individual variations.

### Experimental Example 7: Pharmacokinetic study

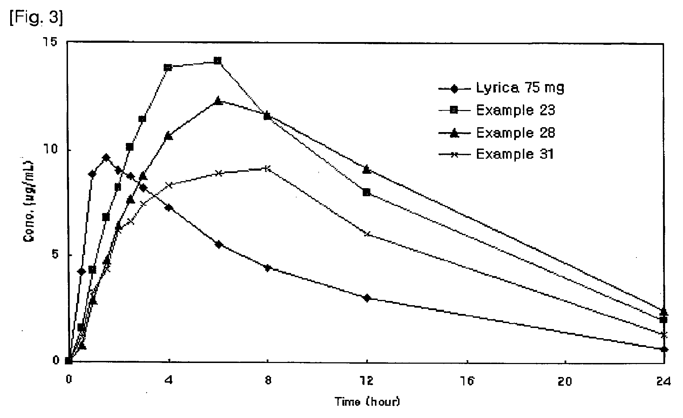

The pharmacokinetic studies on the tablets prepared in Examples 5, 13, 20, 23, 28, and 31 were performed using beagle dogs, according to the same method as in Example 6. The commercially available Lyrica™ Capsule 150 mg (Pfizer Inc.) and Lyrica™ Capsule 75 mg (Pfizer Inc.) were used as a comparative formulation. The concentration of pregabalin in the serum was analyzed with LC/MS/MS. The blood concentration profiles are shown in FIGs. 2 and 3; and the pharmacokinetic parameters obtained therefrom are presented in Tables 20 and 21 below.

**<Table 20>**

| Parameters | Comparative formulation (Lyrica Cap. 150 mg) | Example 5 | Example 13 | Example 20 |
|---|---|---|---|---|
| AUC₀₋₂₄ₕᵣ (ug · hr/mL) | 156.1 | 319.3 | 306.1 | 227.5 |
| Cmax (ug/mL) | 22.0 | 27.2 | 22.9 | 18.6 |
| Tmax (hr) | 0.7 | 5.0 | 6.5 | 4.8 |

**<Table 21>**

| Parameters | Comparative formulation (Lyrica Cap. 75 mg) | Example 23 | Example 28 | Example 31 |
|---|---|---|---|---|
| AUC₀₋₂₄ₕᵣ (ug · hr/mL) | 89.4 | 184.3 | 181.5 | 130.9 |
| Cmax (ug/mL) | 10.8 | 14.9 | 12.3 | 9.9 |
| Tmax (hr) | 1.5 | 5.0 | 6.0 | 5.5 |

As shown in FIG. 2 and Table 20, the absorptions of the sustained release tablets of Examples 5, 13, and 20 were more delayed, in comparison with the immediate release tablet of the comparative formulation (Lyrica™ Capsule 150 mg). And also, the tablets of Examples 5, 13, and 20 showed delayed Tmax values, unlike the comparative formulation for twice-a-day administration; and about 2.0 times, 1.9 times, and 1.5 times higher AUC₀₋₂₄ₕᵣ than the comparative formulation, respectively. Similarly, as shown in FIG. 3 and Table 21, the absorptions of the sustained release tablets of Examples 23, 28, and 31 were more delayed, in comparison with the immediate release tablet of the comparative formulation (Lyrica™ Capsule 75 mg). And also, the tablets of Examples 23, 28, and 31 showed delayed Tmax values; and about 2.1 times, 2.0 times, and 1.5 times higher AUC₀₋₂₄ₕᵣ than the comparative formulation, respectively. Considering that pregabalin is absorbed in the upper small intestine, the sustained release tablet of the present invention can stay in the stomach for longer period than the comparative formulation; and the amount absorbed can be controlled. And also, it can be seen that, even when the dose is changed, the resulting tablet can stay in the stomach for long period; and therefore allow for sustained release of pregabalin.

## Claims

1. A sustained release tablet having two-phase release-controlling system, which consists of a first release-controlling phase comprising pregabalin or its salt and hydroxypropyl methylcellulose; and a second release-controlling phase comprising polyethylene oxide as a swelling polymer, the first release-controlling phase being homogeneously dispersed in the second release-controlling phase.

2. The sustained release tablet according to claim 1, wherein the hydroxypropyl methylcellulose in the first release-controlling phase has a viscosity ranging from 100 to 150,000 centipoises.

3. The sustained release tablet according to claim 2, wherein the hydroxypropyl methylcellulose is present in an amount ranging from 10 to 70 % by weight, based on the total weight of the tablet.

4. The sustained release tablet according to claim 1, wherein the polyethylene oxide in the second release-controlling phase has an average molecular weight ranging from 100,000 to 7,000,000.

5. The sustained release tablet according to claim 4, wherein the polyethylene oxide is present in an amount ranging from 5 to 40 % by weight, based on the total weight of the tablet.

6. The sustained release tablet according to claim 1, wherein the first release-controlling phase further comprises hydroxypropyl cellulose as a binder.

7. The sustained release tablet according to claim 1, wherein the second release-controlling phase further comprises crospovidone or sodium starch glycolate as a supplemental floating agent.

8. The sustained release tablet according to any one of claims 1 to 7, wherein a time to reach maximum plasma concentration (Tmax) ranges from 4 to 8 hours after oral administration.

9. The sustained release tablet according to any one of claims 1 to 7, wherein the tablet is magnified to 12 mm of size when contacting with water.

10. The sustained release tablet according to any one of claims 1 to 7, wherein the tablet is floated within 30 minutes after contact with water and the resultant floating state is maintained for at least 12 hours.

11. A process for preparing a sustained release tablet having two-phase release-controlling system, the process comprising (a) granulating a mixture of pregabalin or its salt and hydroxypropyl methylcellulose; and (b) mixing the granules obtained in the step (a) with polyethylene oxide and a pharmaceutically acceptable excipient, followed by compressing the resulting mixture.

12. The process for preparing a sustained release tablet having two-phase release-controlling system according to claim 11, wherein the granulating is performed using a binder solution.

## Patentansprüche

1. Tablette mit retardierter Freisetzung, die ein Zwei-Phasen-Freisetzungssteuerungssystem aufweist, das aus einer ersten Freisetzungssteuerungsphase, die Pregabalin oder dessen Salz und Hydroxypropylmethylcellulose umfasst; und einer zweiten Freisetzungssteuerungsphase, die Polyethylenoxid als ein quellendes Polymer umfasst, besteht, wobei die erste Freisetzungssteuerungsphase homogen in der zweiten Freisetzungssteuerungsphase dispergiert ist.

2. Tablette mit retardierter Freisetzung nach Anspruch 1, wobei die Hydroxypropylmethylcellulose in der ersten Freisetzungssteuerungsphase eine Viskosität hat, die von 100 bis 150.000 Centipoise reicht.

3. Tablette mit retardierter Freisetzung nach Anspruch 2, wobei die Hydroxypropylmethylcellulose in einer Menge vorliegt, die von 10 bis 70 Gew.-% reicht, bezogen auf das Gesamtgewicht der Tablette.

4. Tablette mit retardierter Freisetzung nach Anspruch 1, wobei das Polyethylenoxid in der zweiten Freisetzungssteuerungsphase ein durchschnittliches Molekulargewicht hat, das von 100.000 bis 7.000.000 reicht.

5. Tablette mit retardierter Freisetzung nach Anspruch 4, wobei das Polyethylenoxid in einer Menge vorliegt, die von 5 bis 40 Gew.-% reicht, bezogen auf das Gesamtgewicht der Tablette.

6. Tablette mit retardierter Freisetzung nach Anspruch 1, wobei die erste Freisetzungssteuerungsphase weiterhin Hydroxypropylcellulose als ein Bindemittel umfasst.

7. Tablette mit retardierter Freisetzung nach Anspruch 1, wobei die zweite Freisetzungssteuerungsphase weiterhin Crospovidon oder Natrium-Stärke-Glykolat als ein zusätzliches Schwemmmittel umfasst.

8. Tablette mit retardierter Freisetzung nach einem der Ansprüche 1 bis 7, wobei eine Zeit zum Erreichen einer maximalen Plasmakonzentration (Tmax) von 4 bis 8 Stunden nach einer oralen Verabreichung reicht.

9. Tablette mit retardierter Freisetzung nach einem der Ansprüche 1 bis 7, wobei die Tablette bei Kontakt mit Wasser auf eine Größe von 12 mm vergrößert wird.

10. Tablette mit retardierter Freisetzung nach einem der Ansprüche 1 bis 7, wobei die Tablette innerhalb von 30 Minuten nach einem Kontakt mit Wasser an die Oberfläche geschwemmt wird und der resultierende Schwemmzustand für mindestens 12 Stunden aufrechterhalten wird.

11. Verfahren zur Herstellung einer retardierten Tablette, die ein Zwei-Phasen-Freisetzungssteuerungssystem aufweist, wobei das Verfahren (a) das Granulieren eines Gemischs von Pregabalin oder dessen Salz und Hydroxypropylmethylcellulose und (b) das Mischen des im Schritt (a) erhaltenen Granulats mit Polyethylenoxid und einem pharmazeutisch akzeptablen Hilfsstoff, gefolgt von dem Verpressen des resultierenden Gemischs umfasst.

12. Verfahren zur Herstellung einer retardierten Tablette, die ein Zwei-Phasen-Freisetzungssteuerungssystem aufweist, nach Anspruch 11, wobei das Granulieren unter Verwendung einer Bindemittellösung durchgeführt wird.

## Revendications

1. Comprimé à libération continue possédant un système de commande de libération à deux phases, constitué d'une première phase de commande de libération comprenant de la prégabaline ou un de ses sels et de l'hydroxypropyl-méthylcellulose ; et d'une deuxième phase de commande de libération comprenant du polyoxyde d'éthylène en tant que polymère gonflant, la première phase de commande de libération étant dispersée de manière homogène dans la deuxième phase de commande de libération.

2. Comprimé à libération continue selon la revendication 1, dans lequel l'hydroxypropylméthylcellulose dans la première phase de commande de libération possède une viscosité allant de 100 à 150 000 centipoises.

3. Comprimé à libération continue selon la revendication 2, dans lequel l'hydroxypropylméthylcellulose est présente en une quantité allant de 10 à 70 % en poids, rapportée au poids total du comprimé.

4. Comprimé à libération continue selon la revendication 1, dans lequel le polyoxyde d'éthylène dans la deuxième phase de commande de libération possède une masse moléculaire moyenne allant de 100 000 à 7 000 000.

5. Comprimé à libération continue selon la revendication 4, dans lequel le polyoxyde d'éthylène est présent en une quantité allant de 5 à 40 % en poids, rapportée au poids total du comprimé.

6. Comprimé à libération continue selon la revendication 1, dans lequel la première phase de commande de libération comprend en outre de l'hydroxypropylcellulose en tant que liant.

7. Comprimé à libération continue selon la revendication 1, dans lequel la deuxième phase de commande de libération comprend en outre de la crospovidone ou du glycolate d'amidon sodique en tant qu'agent de flottement supplémentaire.

8. Comprimé à libération continue selon l'une quelconque des revendications 1 à 7, dans lequel le temps pour atteindre la concentration plasmatique maximale (Tmax) se situe dans la plage de 4 à 8 heures après l'administration par voie orale.

9. Comprimé à libération continue selon l'une quelconque des revendications 1 à 7, dans lequel la taille du comprimé augmente jusqu'à 12 mm lorsqu'il est mis en contact avec de l'eau.

10. Comprimé à libération continue selon l'une quelconque des revendications 1 à 7, dans lequel le comprimé flotte dans les 30 minutes suivant sa mise en contact avec de l'eau et l'état flottant ainsi obtenu est maintenu pendant au moins 12 heures.

11. Procédé de préparation d'un comprimé à libération continue possédant un système de commande de libération à deux phases, le procédé comprenant (a) la granulation d'un mélange de prégabaline ou d'un de ses sels et d'hydroxypropylméthylcellulose ; et (b) le mélangeage des granulés obtenus à l'étape (a) avec du polyoxyde d'éthylène et un excipient pharmaceutiquement acceptable, suivi de la compression du mélange ainsi obtenu.

12. Procédé de préparation d'un comprimé à libération continue possédant un système de commande de libération à deux phases selon la revendication 11, dans lequel la granulation se fait en utilisant une solution de liant.
